# EUROPEAN PATENT SPECIFICATION

(11) **EP 0 861 641 B1**
(45) Date of publication and mention of the grant of the patent: **07.05.2003**
(21) Application number: 97109229.1
(22) Date of filing: 06.06.1997
(51) Int. Cl.: A61F 13/15

(54) **Multi-layer sanitary napkin**
Mehrschichtige Damenbinde
Serviette hygiénique multicouche

(30) Priority: 17.02.1997 JP 4692897
(43) Date of publication of application: 02.09.1998
(73) Proprietor: Asbe Co., Ltd., Tokyo 104 (JP)
(72) Inventor: Yamada, Ikudo, Chou-ku, Tokyo 104 (JP)
(74) Representative: Finsterwald, Manfred, Dipl.-Ing., Dipl.-Wirtsch.-Ing.

(56) References cited:
- EP-A- 0 788 785
- WO-A-95/29655
- US-A- 2 929 379
- US-A- 5 429 631

## Description

### BACKGROUND OF THE INVENTION

The field of art to which the invention pertains includes "bandage, covering articles or absorbing pads for physiological body-liquids such as urine or blood" as contained in IPC A61L 15/16.

### STATE OF THE ART

Absorbing pads are used to treat woman's physiological discharges of menses or leukorrhea. Though the menses pad and leukorrhea pad are similarly structured, the latter is smaller than the former, having the length and width approximately half compared to those of the former. The former is used in a menses period, while the use of the latter is not restricted to a particular period, though the necessity thereof is different from person to person.

In the market, the menses pad and leukorrhea pad are often distinguished from each other by calling the former a "sanitary napkin" and the latter a "flows napkin", for instance. However, in this description, both are generally referred to as "sanitary napkin"s.

Usually, sanitary napkins for menses or leukorrhea are supplied in a single-use form (hereinafter referred to as "ordinary napkins). With an ordinary napkin, a woman is obliged to renew it frequently, and carry replacement napkins always with her in a pouch or something, which may cause her inconveniences and reservations of public notice.

The ordinary napkin comprises a waterproof sheet with a sticking agent applied to the back thereof, an absorbing layer made of water-absorbent fibrous material attached to the waterproof sheet, and a fine mesh screen covering the absorbing layer.

The waterproof sheet avoids seepage of moisture to the rear of the napkin, while the sticking agent sets the napkin onto the underwear surface to prevent it from slipping off. Menses blood or leukorrhea liquid is absorbed by the absorbing layer through the fine mesh screen that serves as an absorbing surface next to the skin. The fine mesh screen restrains the backward flow of liquids to improve the touch to the skin.

Recent advances in the materials science have enabled water-absorbent polymers to attain a very high absorption capability, which benefits the ordinary napkins in both aspects of enhancing the absorption capacity per napkin and lessening the thickness thereof. However, the direction of enhancing the absorption capacity is left within a certain level because of accompanying hygienic difficulties. Therefore, circumstances remain unchanged in which the ordinary napkin needs to be replaced with new one before an absorption limit is reached.

US-A-2 929 379 discloses a multi-layer sanitary napkin in accordance with the preamble of claim 1.

WO 95 29655 A discloses a multilayered sanitary napkin having a plurality of absorbent pads. The first, lowermost pad in the product is configured for releasable attachment to a user's garment. Additional pads in the product are releasably attached above the first pad to form a stacked or layered product.

### SUMMARY OF THE INVENTION

The objective of the invention is to present another type of sanitary napkins for menses or leukorrhea which need not be carried explicitly for replacement, to lessen to some extent the aforementioned woman's mental burdens in relation to her physiological phenomena.

To attain the above objective, the present invention resides in a multi-layer sanitary napkin in which a plurality of ordinary napkins are piled with respective absorbing surfaces thereof directed upward and peelably joined with each other into unity at rims of the ordinary napkins.

In the above multi-layer sanitary napkin (hereinafter referred to as an "invented napkin"), 3 to 6 ordinary napkins for example, are piled and joined into unity. The idea of the invented napkin has arisen from the fact that today's ordinary napkins are so thin, less than 2 mm thick for example, that duplication or triplication of them in use can be done without any trouble.

One who wears the invented napkin can wear a bundle of ordinary napkins (hereinafter simply referred to as "piece"s) for almost one day's consumption. When the uppermost piece approaches the absorption limit thereof, it is peeled off from the invented napkin and discarded, to expose the next absorbing surface for use.

To produce the invented napkin, it is necessary to join the pieces with each other in such a manner that each piece can be peeled off one after another. For joining the pieces in such manner, bonding the rims of the pieces together with a peelable bonding agent, embossing the rims of piled-up pieces between a pair of molds to produce a peelable mechanical connection between the rims, or simultaneously applying the both, are available.

The form of the invented napkin raised above can be realized by piling the pieces with the absorbing surfaces thereof pressed downward onto a mould having a convex surface, and joining the pieces together by bonding, embossing, or bonding and embossing simultaneously. Heat may also be applied at a temperature between a softening point and a melting point of the materials of the piece, specifically, those of the waterproof sheet.

Different from the above form, if the pieces are piled and joined while the absorbing surfaces are kept flat, wrinkles can be formed on the top surface when the invented napkin is bent along the body's figure, which may cause a side leakage.

It is preferable that the invented napkin is provided with pull tabs on either end of respective pieces, each tab being formed not so as to overlap on neighbouring ones.

By pulling the pull tab raised above with fingers, the uppermost piece can easily be peeled off from the invented napkin. Each pull tab is formed not so as to overlap on neighboring ones, to make it easier than otherwise to hold the pull tab with fingers. The pull tabs are formed on either the front or rear end of respective pieces, because it is convenient to form the pull tabs in these spaces.

When a woman wears the invented napkin, she becomes free from carrying the replacement napkins and her mental burdens. Since dirtied napkins are peeled off one after another and treated every few hours, cleanliness and good skin touch can be maintained.

### BRIEF DESCRIPTION OF THE DRAWING

The present invention can be further understood by reference to the following description and attached drawing which illustrates a preferred embodiment.

In the drawing:
Fig. 1 shows a longitudinal cross-sectional view of a multi-layer sanitary napkin according to the embodiment;
Fig. 2 shows a lateral cross-sectional view of the multi-layer sanitary napkin seen in the direction of arrows II-II' drawn in Fig. 1; and
Fig. 3 shows a partial plane view of the multi-layer sanitary napkin seen in the direction of arrows III-III' drawn in Fig. 1.

### DESCRIPTION OF THE PREFERRED EMBODIMENT

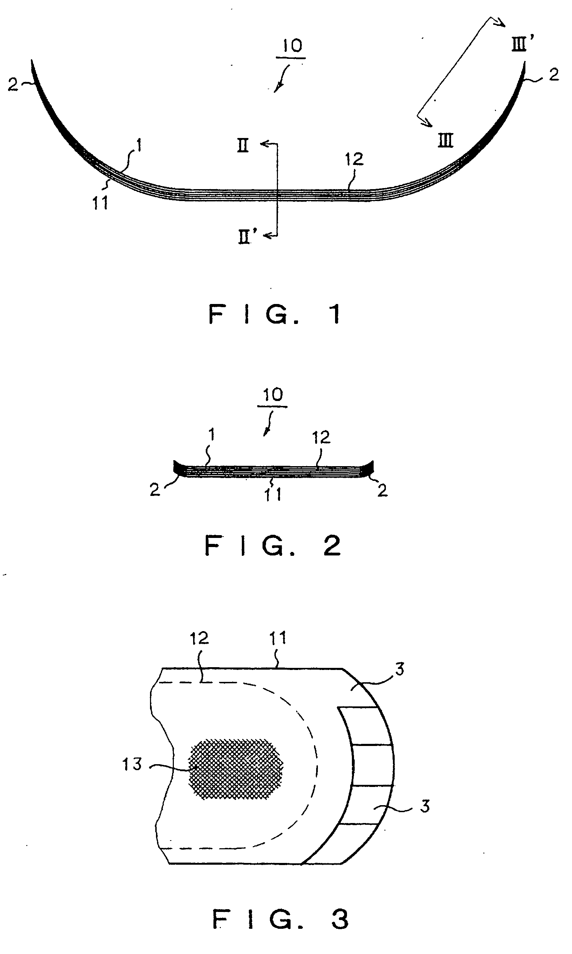

Figs. 1 and 2 in combination show the construction of a multi-layer sanitary napkin **10** according to a preferable embodiment of the invention, in which reference numeral **1** denotes an ordinary napkin constituting the multi-layer sanitary napkin **10**, **11** a waterproof sheet, **12** an absorbing layer, **13** a fine mesh screen (see Fig. 3), **2** a junction layer formed on the rim of the multi-layer sanitary napkin **10**, and **3** a pull tab formed on one end of each ordinary napkin **1**, respectively.

As seen in Figs. 1 and 2, the multi-layer sanitary napkin **10** comprises a plurality of the ordinary napkins **1** which are piled one on top of the other, with respective absorbing surfaces thereof directed upward, and peelably joined with each other into unity, at rims of the ordinary napkins **1**. In the multi-layer sanitary napkin **10**, respective absorbing surfaces of the ordinary napkins **1** are shaped as concave as a ship's bottom, so that the entire figure of the multi-layer sanitary napkin **10** is shaped in the same manner.

The multi-layer sanitary napkin **10** shaped concave in the above-mentioned manner is produced by applying a peelable bonding agent to the rim of each ordinary napkin **1**, piling the ordinary napkins **1** with respective absorbing surfaces thereof directed downward on a mould having a convex surface like a ship's bottom, and embossing the rims of the piled-up ordinary napkins **1** with another mold while applying pressure and heat.

Since the multi-layer sanitary napkin **10** thus produced has been shaped in advance to fit the body's figure, break-down wrinkles are hard to be formed on the top surface thereof when it is worn for use. A side leakage is effectively prevented and a good touch to the skin is given.

When the ordinary napkin **1** on top of the multi-layer sanitary napkin **10** approaches the absorption limit thereof, it can be peeled off to expose the next absorbing surface for use by pulling the pull tab 3 shown in Fig. 3.

## Claims

1. A multi-layer sanitary napkin (10) comprising a plurality of ordinary napkins (1) for treating menses or leukorrhea in a single use, each ordinary napkin (1) having an absorbing surface (12) and a rim, said ordinary napkins (1) being piled on one another with respective absorbing surfaces (12) thereof directed upward,
**characterized in that**
said absorbing surfaces (12) are longitudinally and cross-sectionally concave and said ordinary napkins (1) are peelably joined with each other into unity at said rims.

2. A multi-layer sanitary napkin according to claim 1, further comprising pull tabs (3) formed on either end of respective said ordinary napkins (1), wherein each of said pull tabs (3) is formed not so as to overlap neighbouring ones.

3. A multi-layer sanitary napkin according to claim 1, wherein said rims are embossed between a pair of moulds to produce a peelable mechanical connection between said rims.

4. A multi-layer sanitary napkin according to claim 3, wherein heat is applied during said embossing at the temperature between a softening point and a melting point of materials constituting said ordinary napkin.

## Patentansprüche

1. Mehrlagige Hygienebinde (10) mit mehreren gewöhnlichen Binden (1) zur Behandlung von Monatsblutungen oder Leukorrhö mit einmaligem Gebrauch, wobei jede gewöhnliche Binde (1) eine absorbierende Oberfläche (12) und einen Randbereich aufweist, wobei die gewöhnlichen Binden (1) übereinander gelegt und deren jeweilige absorbierende Oberflächen (12) nach oben gerichtet sind,
**dadurch gekennzeichnet,**
**dass** die absorbierenden Oberflächen (12) in Längsrichtung und im Querschnitt konkav sind, und die gewöhnlichen Binden (1) an den Randbereichen miteinander zu einer Einheit abschälbar zusammengefügt sind.

2. Mehrlagige Hygienebinde nach Anspruch 1, die ferner Zuglaschen (3) umfasst, die an einem oder beiden Enden der jeweiligen gewöhnlichen Binden (1) ausgebildet sind, wobei eine jede der Zuglaschen (3) derart ausgebildet ist, dass sie eine benachbarte Zuglasche nicht überlappt.

3. Mehrlagige Hygienebinde nach Anspruch 1, wobei die Randbereiche zwischen zwei Formen geprägt sind, um zwischen den Randbereichen eine abschälbare mechanische Verbindung herzustellen.

4. Mehrlagige Hygienebinde nach Anspruch 3, wobei während des Prägens Wärme aufgebracht wird, wobei die Temperatur zwischen einem Erweichungspunkt und einem Schmelzpunkt von Materialien, die die gewöhnliche Binde bilden, liegt.

## Revendications

1. Serviette hygiénique multicouche (10) comprenant une pluralité de serviettes classiques (1) pour flux menstruels ou leucorrhée à la fois, chaque serviette classique (1) comportant une surface absorbante (12) et une bordure, lesdites serviettes classiques (1) étant superposées avec leur surface absorbante (12) respective donnant vers le haut,
**caractérisée en ce que**
lesdites surfaces absorbantes (12) sont concaves longitudinalement et en coupe transversale et lesdites serviettes classiques (1) sont reliées avec possibilité de séparation les unes aux autres en un tout auxdites bordures.

2. Serviette hygiénique multicouche selon la revendication 1, comprenant également des pattes à détacher (3) formées sur l'une ou l'autre extrémité desdites serviettes classiques (1), dans laquelle chacune desdites pattes à détacher (3) est formée de manière à ne pas recouvrir les pattes voisines.

3. Serviette hygiénique multicouche selon la revendication 1, dans laquelle lesdites bordures sont gaufrées entre une paire de moules pour produire une connexion mécanique détachable entre lesdites bordures.

4. Serviette hygiénique multicouche selon la revendication 3, dans laquelle de la chaleur est appliquée durant ledit gaufrage à la température entre un point de ramollissement et un point de fusion des matériaux constituant ladite serviette classique.
